(19) Europäisches Patentamt
European Patent Office
Office européen des brevets

(11) Veröffentlichungsnummer: **0 251 110 B1**

(12) **EUROPÄISCHE PATENTSCHRIFT**

(45) Veröffentlichungstag der Patentschrift: **10.04.91**

(51) Int. Cl.5: **C08G 73/12**, C08G 65/32, C08L 79/08, C08L 63/10, C08L 67/06, C07D 401/12, C07D 207/44

(21) Anmeldenummer: **87108937.1**

(22) Anmeldetag: **23.06.87**

# (54) Flexible Bismaleinimide.

(30) Priorität: **02.07.86 DE 3622088**

(43) Veröffentlichungstag der Anmeldung: **07.01.88 Patentblatt 88/01**

(45) Bekanntmachung des Hinweises auf die Patenterteilung: **10.04.91 Patentblatt 91/15**

(84) Benannte Vertragsstaaten: **CH DE FR GB IT LI**

(56) Entgegenhaltungen:
**DE-A- 2 019 396**
**DE-A- 2 715 503**
**US-A- 4 116 937**
**US-A- 4 575 542**

(73) Patentinhaber: **BASF Aktiengesellschaft**
**Carl-Bosch-Strasse 38**
**W-6700 Ludwigshafen(DE)**

(72) Erfinder: **Eisenbarth, Philipp., Dr.**
**Gutleutstrasse 12**
**W-6702 Bad Duerkheim(DE)**
Erfinder: **Hesse, Anton, Dr.**
**Peter-Nickel-Strasse 15**
**W-6940 Weinheim(DE)**
Erfinder: **Holoch, Jan, Dr.**
**Helaweg 11**
**W-6900 Heidelberg(DE)**
Erfinder: **Peter, Roland, Dr.**
**Wildermuthstrasse 33**
**W-6700 Ludwigshafen(DE)**

**EP 0 251 110 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft flexible Bismaleinimide, die durch Umsetzung von Maleinsäureanhydrid mit einem aminoterminierten Polyyoxybutylen hergestellt werden.

Bismaleinimidharze sind heißhärtbare Reaktionsharze, die z.B. in Form von gefüllten und ungefüllten Preßmassen sowie faserverstärkten Prepregs eine zunehmende Anwendung in den Bereichen Luft- und Raumfahrt, im Automobil und Maschinenbau und in der Elektronikindustrie finden. Neben der guten Hitzebeständigkeit und dem hohen E-Modul besitzen diese duromeren Werkstoffe jedoch als wesentlichen Nachteil eine vergleichsweise große Sprödigkeit, deren Ursache in der hohen Vernetzungsdichte liegt. So werden beispielsweise gerade bei Anwendungen als Strukturbauteile für Flugzeuge sehr hohe Anforderungen an die Duktilität und Zähigkeit der eingesetzten Faserverbundwerkstoffe gestellt, die von den derzeit verfügbaren Bismaleinimidharzen noch nicht befriedigend erfüllt werden.

Die zunächst naheliegende Zähmodifizierung von Bismaleinimidharzen mit Kautschuken hat bis heute aus den verschiedensten Gründen noch keinen entscheidenden Durchbruch erbracht. Ein völlig anderes Konzept besteht im Einbau von flexiblen Kettensegmenten in das Polymernetzwerk, indem z.B. gezielt Bismaleinimid-Monomere, deren endständige Maleinimidringe mit einer beweglichen, linearen Molekülkette höheren Molekulargewichts verknüpft sind, eingesetzt werden. Dies wird erfindungsgemäß durch den Einbau von Polyoxybutylensegmenten (Polytetrahydrofuran) erreicht.

Gegenstand der Erfindung sind Bismaleinimide der allgemeinen Formel

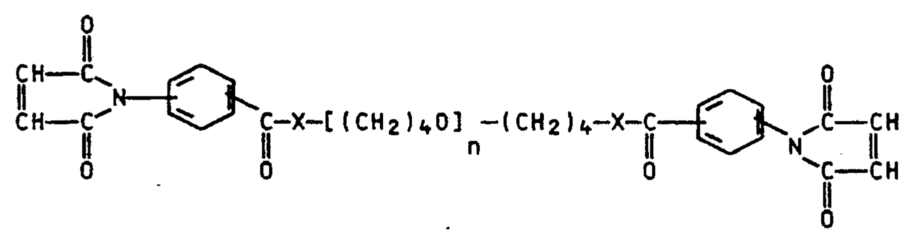

(I)

in der n eine ganze Zahl zwischen 5 und 70 ist, und X für O oder NH steht.

Die erfindungsgemäßen Bismaleinimide werden hergestellt durch Umsetzung von 2 Mol Maleinsäureanhydrid mit einem Mol eines aminoterminierten Polyoxybutylens der Formel

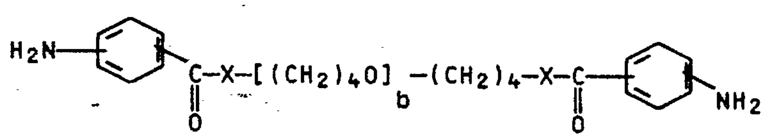

(II)

Die Herstellung von Polyoxybutylen mit aromatischen Aminoendgruppen (II) ist z.B. in der japanischen Offenlegungsschrift JA 59/199 715 beschrieben; man führt eine Umesterung des Polyoxybutylens mit Aminobenzoesäureestern durch (1 Äquiv. pro OH-Gruppe), wobei die niedermolekulare Alkoholkomponente destillativ entfernt wird. Analog verläuft die Umsetzung eines Polyoxybutylens mit aliphatischen Aminogruppen der Formel

$H_2N\text{-}(CH_2)_4\text{-}O\text{-}[(CH_2)_4O]_n\text{-}(CH_2)_4\text{-}NH_2$

mit Aminobenzoesäureestern, wobei Aminobenzamid-Endgruppen gebildet werden. Das Molekulargewicht des eingesetzten Polytetrahydrofurans kann in weiten Grenzen zwischen etwa 400 und etwa 5000 schwanken; bevorzugt ist ein Bereich zwischen 600 bis 2400.

Für die Herstellung der entsprechenden Bismaleinimide der Formel I aus den genannten aminoterminierten Polyoxybutylenen kommen prinzipiell alle bekannten Verfahren zur Herstellung von Bismaleinimiden aus Diaminen in Frage.

Im ersten Reaktionsschritt erfolgt die Umsetzung des aminoterminierten Polyoxybutylens mit Maleinsäureanhydrid in einem aprotischen, polaren organischen Lösungsmittel, wie z.B. Aceton, Methylethylketon, Diethylketon, Cyclohexanon, Dimethylformamid, Dimethylacetamid oder N-Methylpyrrolidon, bei Temperaturen von -30 bis + 50 °C, vorzugsweise von -10 bis + 30 °C. Zweckmäßigerweise werden 1 bis 1,5 Äquivalente Maleinsäureanhydrid pro Aminogruppe eingesetzt. Die dabei zunächst entestehende Bisamidocarbonsäure-Zwischenstufe braucht nicht isoliert zu werden, sondern kann direkt im nächsten Reaktionsschritt unter Wasserabspaltung in das Bismaleinimid überführt werden. Die Dehydratisierung der Bisamidocarbonsäure erfolgt in Gegenwart eines niedermolekularen Carbonsäureanhydrids, vorzugsweise

Acetanhydrid bei Temperaturen zwischen 40 bis 80 °C. Zusätzlich können als Cyclisierungskatalysatoren Metallsalze sowie tertiäre Amine zugegeben werden. Besonders aktive Metallverbindungen sind die Nitrate, Halogenide, Acetate, Acetylacetonate oder Alkoxide von Lithium, Natrium, Magnesium, Calcium, Titan, Nickel, Mangan und Kobalt. Bevorzugt sind die entsprechenden Übergangsmetallsalze. Als tertiäres Amin eignet sich besonders Tiethylamin.

Zur Isolierung des Produktes ist es zweckmäßig, das Lösungsmittel unter leichtem Erwärmen und Anlegen von Vakuum weitgehend abzudestillieren und den öligen Rückstand mit viel Wasser von der gebildeten Essigsäure sowie vom Katalysator zu befreien. Zur besseren Phasentrennung beim Waschvorgang, der mehrmals bis zum Erreichen eines pH-Wertes von ca. 7 wiederholt werden muß, ist eine Verdünnung der organischen Phase mit einem mit Wasser nicht mischbaren Lösungsmittel, z.B. Dichlormethan vorteilhaft. Nach Beendigung des Waschvorgangs wird die organische Phase getrocknet und das Lösungsmittel im Vakuum entfernt. Das Produkt fällt als viskoses, gelbliches bis braunes Öl an, das im Falle eines höheren Molekulargewichts wachsartig erstarrt bzw. kristallisiert.

Die Identität des Produktes ergibt sich aus den IR- und NMR-spektroskopischen Daten sowie aus der Bestimmung der Doppelbindungswerte, die in der Regel zwischen 80 bis 95 % der Theorie betragen; die Löslichkeit der Bismaleinimide ist in vielen organischen Lösungsmitteln, wie z.B. Aceton, Dichlormethan sehr hoch.

Die erfindungsgemäßen Bismaleinimide eignen sich insbesondere als flexibilisierende Zusätze für Bismaleinimidharze, aber auch für ungesättigte Polyesterharze, Vinylesterharze, Cyanatesterharze oder als Vernetzer für Lacke und Klebstoffe. Sie lassen sich auch alleine radikalisch unter Bildung von transparenten, hochflexiblen Formstoffen härten.

Flexible Formstoffe werden erhalten, wenn man ungesättigte Polyesterharze, Vinylesterharze oder Cyanatesterharze aushärtet, die 1 bis 100, vorzugsweise 5 bis 50 Gew.%, bezogen auf das Harz, der erfindungsgemäßen Bismaleinimide enthalten.

Ungesättigte Polyester sind Polykondensationsprodukte aus mehrwertigen, insbesondere zweiwertigen Carbonsäuren bzw. deren veresterbaren Derivaten, vorzugsweise Anhydriden, die mit mehrwertigen, insbesondere zweiwertigen Alkoholen esterartig verknüpft sind, wobei zumindest ein Teil der Reste, bevorzugt die Dicarbonsäurereste, über ethylenisch ungesättigte copolymerisierbare Gruppen verfügen muß. Lösungen dieser Polyester in copolymerisierbaren Monomeren bezeichnet man als ungesättigte Polyesterharze.

Endständig ungesättigte Vinylester im Sinne der Erfindung besitzen die charakteristische Gruppierung $-CO-OCH_2CHOH-CH_2O-$ und enthalten endständige polymerisierbare ungesättigte Gruppen. Die Vinylesterharze werden hergestellt durch Umsetzung von etwa stöchiometrischen Mengen eines Polyexpoxidharzes und einer ungesättigten Monocarbonsäure, beispielsweise Methacrylsäure und Auflösen des Vinylesters in copolymerisierbaren Monomeren.

Cyanatesterharze sind polyfunktionelle Verbindungen, die mindestens zwei Cyanatgruppen im Molekül enthalten, im einfachsten Fall das Umsetzungsprodukt von Bisphenol A mit 2 Mol Chlorcyan. Sie können in trimerisierter Form oder als Vorkondensate mit Aminen, Phenolen oder Epoxiden vorliegen.

Die in den Beispielen genannten Teile und Prozente beziehen sich auf das Gewicht.

Beispiel

a) Herstellung eines Polyoxybutylens mit aromatischen Aminoendgruppen

Eine Mischung aus 3000 g Polytetrahydrofuran mit Molgewicht 650, 1575 g 4-Aminobenzoesäureethylester und 0,45 g Titantetrabutylat wird 22 Stunden auf 200 °C erhitzt, wobei das freigesetzte Ethanol abdestilliert. Nach Abkühlen erhält man das Produkt in Form eines gelben, hochviskosen Öls.

b) Herstellung des Bismaleinimids

Zu einer Lösung von 196 g Maleinsäureanhydrid in 2000 ml Aceton wird bei 0 °C eine Lösung von 890 g des nach a) hergestellten aminoterminierten Polyoxybutylens in 2000 ml Aceton innerhalb 1 Stunde zugetropft. Man rührt noch 2 Stunden ohne Kühlung nach und gibt dann 306 g Acetanhydrid, 60,6 g Triethylamin und 5,88 g Mangan(II)-acetat-tetrahydrat hinzu. Nach 2 Stunden bei 40 °C und 1 Stunde bei 50 °C destilliert man das Aceton im Vakuum weitestgehend ab. Man wäscht zunächst einmal mit 3000 ml Wasser; anschließend gibt man 2500 ml Dichlormethan hinzu und wäscht achtmal mit je 2000 ml Wasser säurefrei. Die organische Phase wird über Natriumsulfat getrocknet und im Vakuum eingedampft. Man erhält 1005 g (96 %) Bismaleinimid als gelbliches, hochviskoses Öl.

c) Flexibilisierung eines ungesättigten Polyesterharzes

250 Teile eines ungesättigten Polyesters aus 101 Teilen Maleinsäure anhydrid, 136 Teilen Dicyclopentadien und 33 Teilen Ethylenglykol werden in 133 Teilen Styrol gelöst. Zu dieser Lösung (Viskosität

170 mPas bei 23 °C) werden 96 Teile (25 %, bezogen auf das Harz) des Bismaleinimids nach b) gegeben.

Nach Zusatz von 2 % Dicumylperoxid werden mit dieser Lösung Glasmatten getränkt. Die Laminate werden in einer Presse bei 150 °C gehärtet. Die Schlagzähigkeit wird nach einem Kugelfalltest (eine Kugel mit Durchmesser 5,7 cm und Masse 0,76 kg wird aus einer Höhe von 75 cm auf den Probekörper fallen gelassen) gemessen. Dabei ergibt sich ein zerstörtes Volumen von 2.800 $mm^3$, während bei Laminaten aus dem Polyesterharz ohne Bismaleinimid-Zusatz sich ein zerstörtes Volumen von über 5.000 $mm^3$ ergeben hatte.

d) Flexibilisierung eines Vinylesterharzes

60 Teile eines Vinylesters aus Bisphenol A-Diepoxid und Methacrylsäure werden in 40 Teilen Styrol gelöst. Dieses Vinylesterharz wird in verschiedenen Verhältnissen mit dem Bismaleinimid nach b) abgemischt.

Nach Zusatz von 0,3 % Methylethylketonperoxid, 1 % Butylperbenzoat und 0,3 % einer 1-%igen styrolischen Lösung von Cobaltoktoat wurden die Harzlösungen in eine Form gegossen. Nach 2 Stunden wurden die plattenförmigen Teile entformt und 2 Stunden bei 150 °C nachgehärtet.

Die Schlagzähigkeit wurde nach DIN 53 453 mit einem Pendelschlagwerk bestimmt.

Ergebnisse

| % zugesetztes Bismaleinimid | Schlagzähigkeit [$kJ \cdot m^{-2}$] |
|---|---|
| 0 | 12 |
| 5 | 15 |
| 10 | 18 |
| 15 | 23 |

## Ansprüche

1. Bismaleinimide der allgemeinen Formel

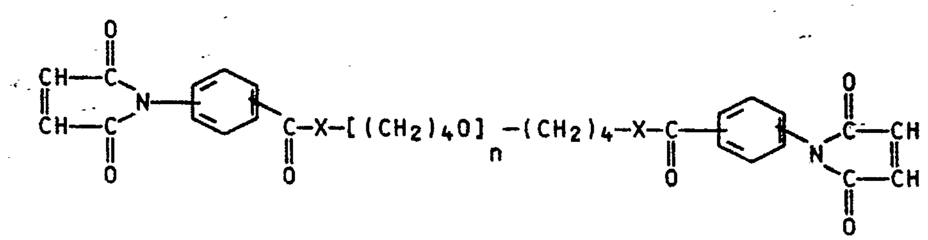

in der n eine ganze Zahl zwischen 5 und 70 ist, und X für O oder NH steht.

2. Verwendung der Bismaleinimide nach Anspruch 1 als flexibilisierende Zusätze zu Bismaleinimidharzen, ungesättigten Polyesterharzen, Vinylesterharzen oder Cyanatesterharzen in Mengen von 1 bis 100 Gew.%, bezogen auf die Harze.

## Claims

1. A bismaleimide of the general formula

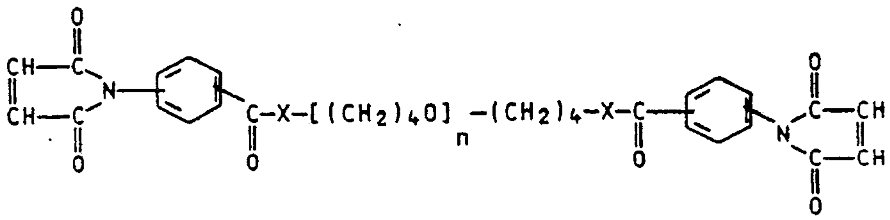

where n is an integer from 5 to 70 and x is 0 or NH.

2. The use of the bismaleimide of claim 1 as a flexibilizing addition to bismaleimide resins, unsaturated polyester resins, vinyl ester resins or cyanate ester resins in an amount of from 1 to 100% by weight, based on the resins.

## Revendications

1. Bismaléimides de formule générale

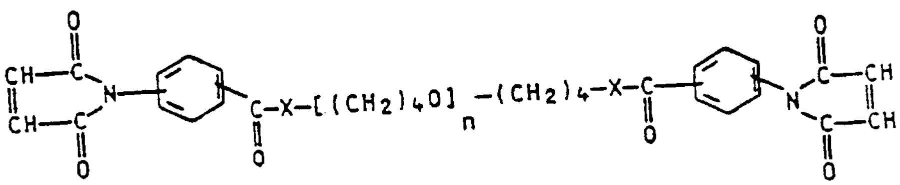

dans laquelle n est un nombre entier compris entre 5 et 70, et X représente O ou NH.

2. Utilisation des bismaléimides suivant la revendication 1 comme additifs conférant de la flexibilité pour des résines de bismaléimides, des résines polyesters insaturées, des résines esters vinyliques ou des résines cyanates (esters), en des quantités de 1 à 100%, en poids par rapport aux résines.